# EUROPEAN PATENT APPLICATION

(11) **EP 2 633 879 A1**
(43) Date of publication of application: **04.09.2013**
(21) Application number: 11835846.4
(22) Date of filing: 27.10.2011
(51) Int. Cl.: A61M 37/00

(54) **MICRO-NEEDLE COATING METHOD**

(30) Priority: 27.10.2010 JP 2010241375; 27.10.2010 JP 2010241376; 27.10.2010 JP 2010241377
(71) Applicant: Medrx Co., Ltd., Kagawa 769-2712 (JP); Teijin Limited, Osaka-shi, Osaka 541-0054 (JP)
(72) Inventor: MAKINO, Yuji, Sanuki-shi Kagawa 769-2193 (JP); KURITA, Takurou, Sanuki-shi Kagawa 769-2193 (JP); HAMAMOTO, Hidetoshi, Higashikagawa-shi Kagawa 769-2712 (JP); KOBAYASHI, Katsunori, Higashikagawa-shi Kagawa 769-2712 (JP)
(74) Representative: Jackson, Martin Peter
(86) International application number: PCT/JP2011/006005
(87) International publication number: WO 2012/056702

(57) **Abstract**

Since all methods of applying a drug liquid to a tip portion of a microneedle are application of the drug liquid in an open system, evaporation of the solvent from the drug liquid is unavoidable and the concentration of the drug liquid changes easily. In addition, a large amount of the drug liquid is necessary. However, since it is difficult to use expensive drugs such as vaccine, protein, antigen and the like in large amounts, a method of applying a drug to a pin-frog-shaped microneedle by using a small amount of the solution and without variation of the concentration of the drug liquid has been desired. A member having plural grooves is produced, and one end thereof is immersed in a drug liquid tank to fill the grooves with the drug liquid. It has been found that the drug can be uniformly applied to a tip portion of the microneedle by setting the pin-frog-shaped microneedle to pass through the drug liquid in the grooves, whereby a pin-frog-shaped microneedle uniformly coated with the drug can be provided.

## Description

### Technical Field

The present invention relates to a method of uniformly coating a microneedle with a drug. Particularly, the present invention relates to a method capable of uniformly coating a tip portion of a pin-frog-shaped (kenzan-shaped) microneedle with a drug.

### Background Art

In recent years, an attempt has been made to transdermally administer a drug using a microneedle, as a new transdermal administration method. While many prior art documents relating to the production methods of microneedles have been published, not many reports teach how to achieve transdermal absorption of a drug.
As a method of carrying a drug on a microneedle, two methods are available: one including mixing a drug in advance in the material of the microneedle and one including applying a drug to the microneedle after production. Of these, the method including applying a drug to the microneedle is superior in broad utility and is also applicable to drugs which are weak to heat and oxidation (proteins such as antigen etc.).
As a method of applying a drug to a microneedle, a method of placing a microneedle on a roller applied with a thin-film containing a drug to coat a tip portion of the microneedle with the drug liquid (roller coating method) is known, as shown in patent documents 1 and 2 and non-patent document 1. In addition, an application method using a dispenser as shown in patent document 3 and an application method using an ink jet technology as shown in patent document 4 are known.

The above-mentioned roller coating method has defects in that solvent easily scatters since a drug liquid is applied on a roller to form a thin film, and the concentration of the drug liquid varies easily. Moreover, when the drug liquid has low viscosity, it does not easily attach to the roller and, unless the rotational frequency of the roller is increased, the drug liquid is not easily held on the top portion of the roller, but when the rotational frequency of the roller is increased, uniform application of the drug liquid to the microneedle becomes difficult. Furthermore, it is difficult to increase the thickness of the drug liquid layer on the roller, which reduces the length of the drug liquid applicable to small needles and limits the application amount. The ink jet method has defects in that control is difficult and the cost of the apparatus itself is high. The dispenser method has been pointed out to have a defect in that the control for application to the tip portion of a microneedle is difficult.
Therefore, a method of coating a microneedle, which is a simple method and requires an economical apparatus, has been desired.

### [Patent List]

### [patent documents]

[patent document 1]US Patent No. 6855372
[patent document 2]JPA 2008-546483
[patent document 3]US Patent No. 5916524
[patent document 4]JPA 2010-131123

[non-patent document 1]Pharmaceutical Research, Vol. 27, No. 2, 303-313 (2009)

### [SUMMARY OF THE INVENTION]

### Problems to be Solved by the Invention

The present invention aims to provide a method capable of applying a drug liquid to a tip portion of a pin-frog-shaped microneedle uniformly at a constant concentration.

### Means of Solving the Problems

As mentioned above, there are some known methods for applying a drug liquid to a microneedle. As a method for continuous application to a microneedle, patent document 1 discloses a method of applying a drug liquid using a roller. In realization of this method, the following problem has emerged. That is, when the drug liquid has low viscosity, the drug liquid attached to a roller immediately runs down and cannot coat a microneedle. When the viscosity is high, the drug liquid tends to make a gathering part on a tip portion of a microneedle due to the surface tension, which in turn blunts the tip portion of the microneedle with the attached drug and lowers its puncturability against the skin. Furthermore, since the method of patent document 1 is application of a drug liquid in an open system, evaporation of the solvent from the drug liquid is unavoidable. Consequently, the concentration of the drug liquid easily changes and control of the viscosity of the drug liquid is difficult.
Therefore, patent document 1 discloses use of a grooved roller, which is considered an attempt to facilitate holding of the drug liquid on the roller. However, the viscosity of the drug liquid in patent document 1 is preferably 20 to 50 cP (centipoise). Since this viscosity is as low as the viscosity of cow milk and juice, an appropriate application of a drug liquid is considered to have become difficult since the drug liquid easily flows out from the grooves of a roller, and the surface of the drug liquid in the grooves in the top portion of the roller decreases. Due to the above, a wide application (in the height direction) to a long needle has been difficult. In addition, the realizability of the suggestion of patent document 1 is also low. To increase the viscosity, not only the drug concentration needs to be increased but also extra additives such as a thickening agent and the like need to be added in large amounts. In transdermal administration, therefore, the extra reagents are intradermally uptaken to easily cause adverse reactions such as skin disorder and the like.

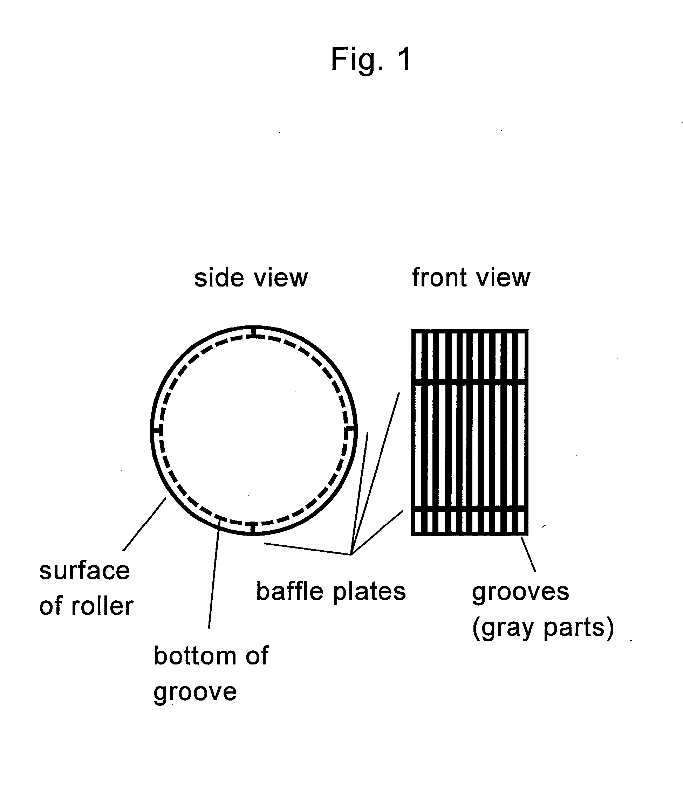

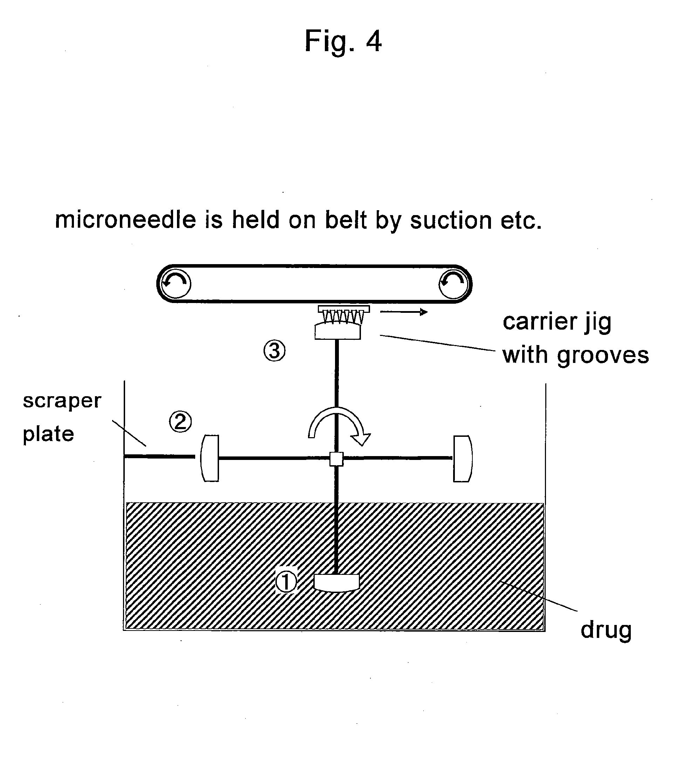

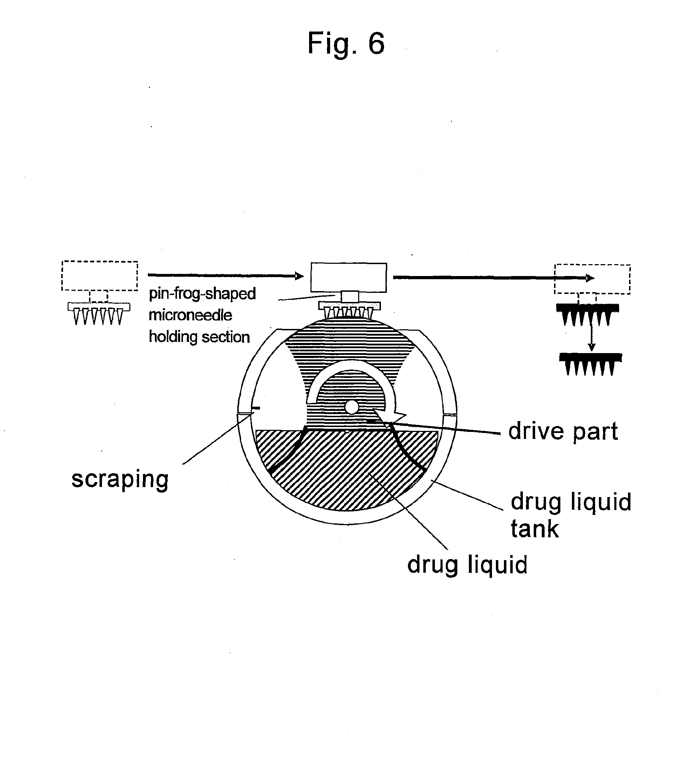

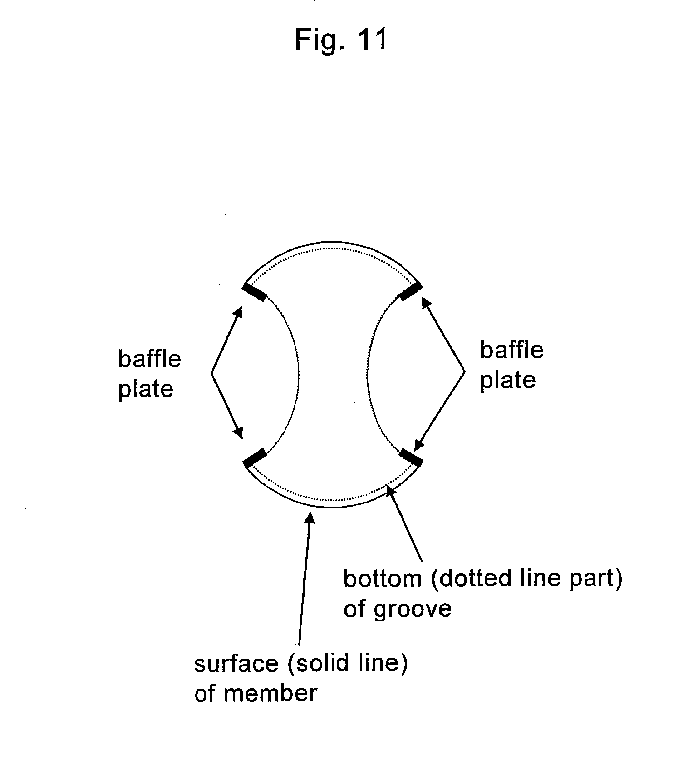

Therefore, the present inventors have considered a method capable of appropriate application regardless of the viscosity of the drug liquid, even when a drug liquid has a low viscosity, and found that the method can be obtained by the following two methods.
a) A roller (cylindrical member) with break(s) in the grooves is used. That is, due to break(s) in the grooves of a roller (when a part of the circumferential portion of the roller is partially missing as shown in Fig. 6, or waterwheel type as shown in Fig. 4 and the like), the drug liquid is held in the break in the grooves by surface tension, and does not easily flow down along the grooves. Hence, the drug liquid tends to easily stay in the grooves. As compared to a method using a simply-grooved roller, therefore, uniform application of a drug liquid can be performed using a microneedle with longer needles, which sets the application position higher (much in the height direction).
b) A roller with grooves having a partition by a shielding plate (baffle plate) is used. That is, it has been found that the movement and flowing out of a drug liquid can be suppressed by using the member of Fig. 1 having a baffle plate placed in the grooves of the roller. In addition, it has also been found that a drug liquid does not flow out easily along the grooves by placing a baffle plate on both ends of the grooves of the member of the above-mentioned a) (member lacking part of roller, for example, member having hourglass shape *(tsuzumi-*shape) or waterwheel shape etc.) as shown in Fig. 11. That is, it has been found that a drug liquid tends to stay in the groove by placing a baffle plate.
By the above-mentioned two methods, the drug liquid level shows smaller changes even when the groove has a large depth. As a result, the drug liquid can be applied uniformly even when the needle of a microneedle has a larger length (needle having higher height).

In some cases, a single application of a drug liquid cannot produce sufficient holding of the drug. Thus, a plurality of application is necessary. In this event, when the above-mentioned roller (cylindrical member) is used, a transport equipment (carrier) of the microneedle needs to be circulated to effect multiple applications. However, for a system for circulating a carrier, a difficult problem is fixing the microneedle on the carrier such that the microneedle always passes in the groove. On the other hand, it has been clarified that the microneedle is easily positioned and fixed when the carrier moves horizontally on a lane, and the tip portion of the microneedle can be set to always pass in the groove. However, when the carrier is horizontally moved, the second application of the drug liquid is performed in the direction of the microneedle running against the rotational direction of the roller. The problem of application while running backwards is that the drug liquid cannot be uniformly applied to the tip portion of the microneedle.

The reason therefor is as follows. When the movement of the microneedle and the rotation of the roller are synchronized, the movements of the drug liquid and the needles in the groove are the same, and apparently, the microneedle looks like being inserted into and drawn up from a still drug liquid. On the contrary, when the microneedle runs opposite, the microneedle pushes its way through the drug liquid and uniform application to the tip portion of the microneedle is difficult.
Therefore, using a member having a missing part in the circumferential portion (member having hourglass shape or waterwheel shape etc.), a microneedle is made to run opposite when the missing part in the circumferential portion comes. As a result, since the tip portion of the microneedle does not pass the groove and the microneedle does not touch the drug liquid. Then, the microneedle can advance in the forward direction again (direction in which the movement of the microneedle and the rotation of the roller are synchronized). Thus, using a member having a missing part in the circumferential portion, uniform multiple application has been enabled by using a carrier with a horizontal movement.

In addition, the surface of a member having grooves was uniformly leveled with a brush, scraper or the like to make the drug liquid levels of the grooves uniform, thus enabling uniform application of the drug liquid to each tip portion of a pin-frog-shaped microneedle.
Furthermore, when a flat plate-like member having grooves is used, a pin-frog-shaped microneedle is set on a transport equipment (carrier), as shown in Fig. 2, so that the tip portion of the microneedle can move to pass through the groove filled with a drug liquid. The moving rate of the tip portion of the microneedle in the grooves was slow so that each tip portion could be uniformly coated with the drug liquid. To increase the speed of the movement of the pin-frog-shaped microneedle, the member having grooves only needs to be moved in accordance with the moving speed of the microneedle. For this end, a movable member having grooves may be used. For example, using a belt having grooves shown in Fig. 3 or the rotational waterwheel-shaped member in Figs. 4 and 6 (partial fragment member of circular cylinder), the speed of the movement of the microneedle could be increased.

Furthermore, the present inventors have found that a drug liquid can be uniformly applied with good quantitativity to a tip portion of each needle of a pin-frog-shaped microneedle by synchronizing the movement of the member having grooves with that of the pin-frog-shaped microneedle. The present inventors have completed the present invention based on these findings.

The summary of the present invention is as follows.
(1) A method of coating a tip portion of a pin-frog-shaped microneedle with a drug liquid, comprising
   a) setting the following pin-frog-shaped microneedle on a transport equipment (carrier),
      said microneedle having needles with a height of 100 to 1000 µm,
      said microneedle having needles with a basal portion having a width of 100 to 1000 µm,
   b) moving the carrier to have a tip portion of the needles of the pin-frog-shaped microneedle pass through the following grooves filled with a drug liquid,
      said grooves having the number and distance corresponding to the row of needles of the microneedle,
      said grooves having a depth of 0.1 - 10 mm,
   c) moving said carrier in synchronization with and in the same direction as the movement of the member having said grooves, and
   d) drying the tip portion of said needles coated with the drug liquid to carry the drug on the tip portion.
(2) The method of the above-mentioned (1), wherein said member having the grooves is flat plate-like, belt-shaped, cylindrically-shaped or cylindrically-shaped with a missing part.
(3) The method of the above-mentioned (2), wherein the cylindrically-shaped member with a missing part has a cross-section with a waterwheel shape, a spindle shape, an hourglass shape, a Ginkgo shape (Japanese icho shape) or a sector shape.
(4) The method of any of the above-mentioned (1) - (3), wherein the surface of said grooves filled with the drug liquid is leveled with a roller or scraper to provide a uniform liquid surface of the grooves.
(5) The method of any of the above-mentioned (1) - (4), comprising applying and drying the drug liquid several times to carry the drug in a desired amount.
(6) The method of any of the above-mentioned (1) - (5), wherein the needle of said microneedle has a height of 100 to 600 µm and said grooves have a depth of 100 to 600 µm.
(7) The method of any of the above-mentioned (1) - (6), wherein said grooves are blocked at the both ends with a shielding plate having the same height as the surface of the grooves.
(8) The method of any of the above-mentioned (1) - (7), wherein the tip portion of said pin-frog-shaped microneedle is set facing downward in the vertical direction.
(9) The method of any of the above-mentioned (1) - (8), wherein said pin-frog-shaped microneedle is made of a thermoplastic resin.
(10) The method of any of the above-mentioned (1) - (9), wherein the thermoplastic resin is a polyglycolic acid resin.

(11) A member having grooves for applying a drug liquid to a pin-frog-shaped microneedle, wherein
   a) said grooves have a width of 300 to 500 µm,
   b) said grooves have a depth of 300 to 500 µm,
   c) said grooves is in the number of 5 to 20 grooves per 1 cm,
   c) the member having grooves has a cylindrical shape or a cylindrical shape with a missing part, and
   d) the cylindrical shape or a cylindrical shape with a missing part has a diameter of 2.5 - 4 cm, and a thickness of 1 - 3 cm.
(12) The member of the above-mentioned (11), wherein said cylindrical shape with a missing part has a cross-section with a waterwheel shape, a spindle shape, an hourglass shape, a Ginkgo shape or a sector shape.
(13) The member of the above-mentioned (11) or (12), wherein said cylindrical shape with a missing part has a cross-section with an hourglass shape.
(14) The member of any of the above-mentioned (11) - (13), wherein said member having grooves is made of stainless steel.
(15) The member of any of the above-mentioned (13) and (14), wherein said grooves have both ends blocked with a shielding plat having the same height as the surface of the grooves.

### Effect of the Invention

The method for applying a drug liquid of the present invention is a method capable of applying the drug liquid by passing a tip portion of a pin-frog-shaped microneedle through grooves filled with the drug liquid. Since a transport equipment (carrier) can be moved slowly or in synchronization with the member having grooves so that a tip portion of each needle can be uniformly coated while passing through the grooves, a drug can be applied to the tip portion of each needle efficiently and quantitatively. In addition, since a member having grooves and a drug liquid are placed in a closed system and only a part that comes into contact with the microneedle can be in an open system, only a small portion of the drug contacts the outside air. Therefore, the concentration of the drug liquid changes only a little, and uniform and quantitative application to the microneedle has been enabled.

### Brief Description of the Drawings

Fig. 1 shows one embodiment of a roller with grooves (cylindrical-shaped member) placed with a baffle plate to be used in the application method of the present invention.

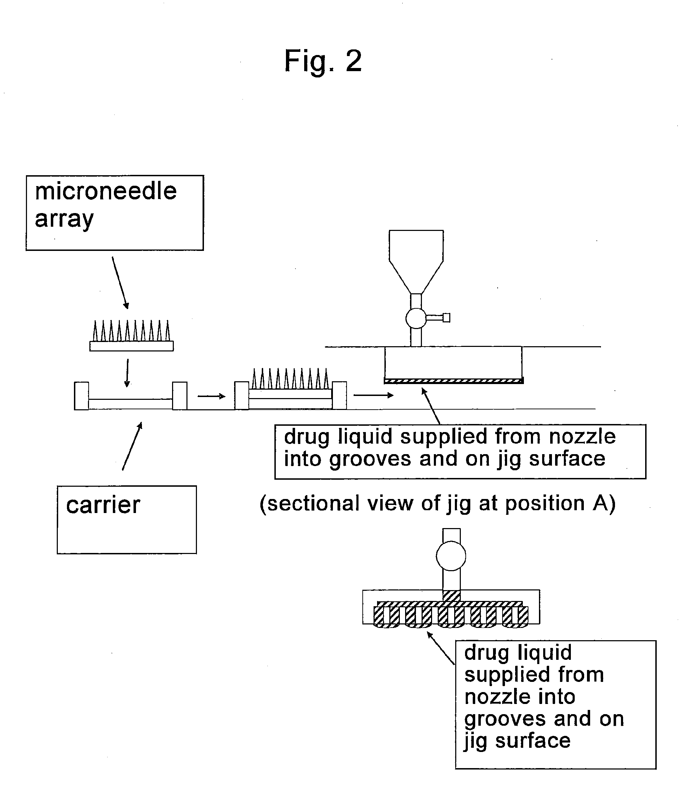

Fig. 2 shows one of the application methods of the present invention, wherein a pin-frog-shaped microneedle is first set on a transport equipment (carrier), and the carrier moves to the direction of the member having grooves.

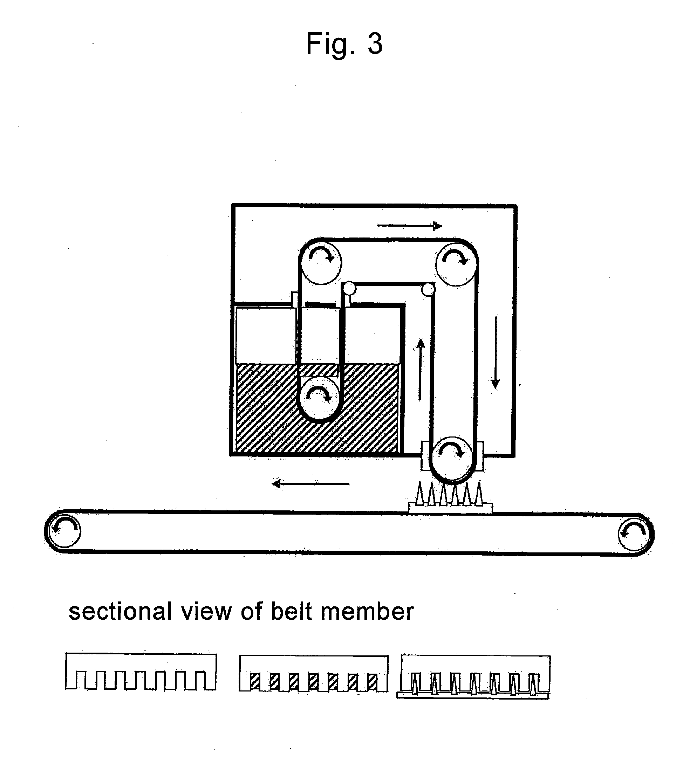

Fig. 3 is a schematic view of one application apparatus using the application method of the present invention wherein the environment of a drug liquid tank and a belt is a closed system to prevent evaporation of a drug liquid. Since a tip portion of the pin-frog-shaped microneedle passes through the grooves of the belt filled with the drug liquid, the tip portion of the microneedle is uniformly coated.
Fig. 4 shows one of the application methods of the present invention wherein a cylindrical waterwheel-shaped member having 4 surface parts is rotated to fill the grooves with the drug liquid, and the pin-frog-shaped microneedle is coated.

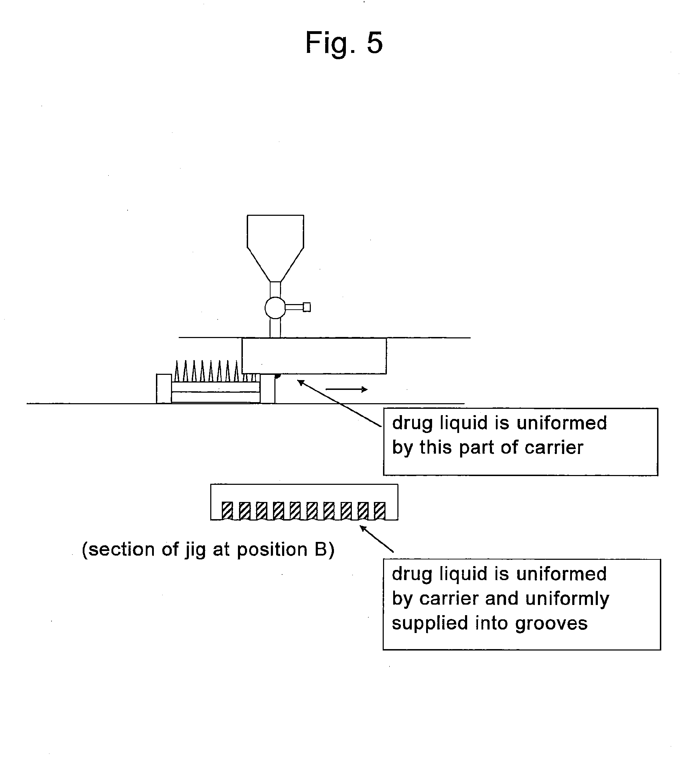

Fig. 5 shows a step following Fig. 2 in the application method of the present invention, which indicates that a rectangular-parallelepiped member set in the front surface in the direction of travel of the carrier advances while scratching the surface of the jig, and acts to flatten the drug liquid, swelling out from the grooves, to the same height as the grooves. This is shown in Fig. 5.
Fig. 6 shows one of the application methods of the present invention, which indicates that an hourglass-shaped member having grooves is used and rotated to fill the grooves with the drug liquid and the microneedle is passed through the grooves to coat a tip portion of the pin-frog-shaped microneedle.

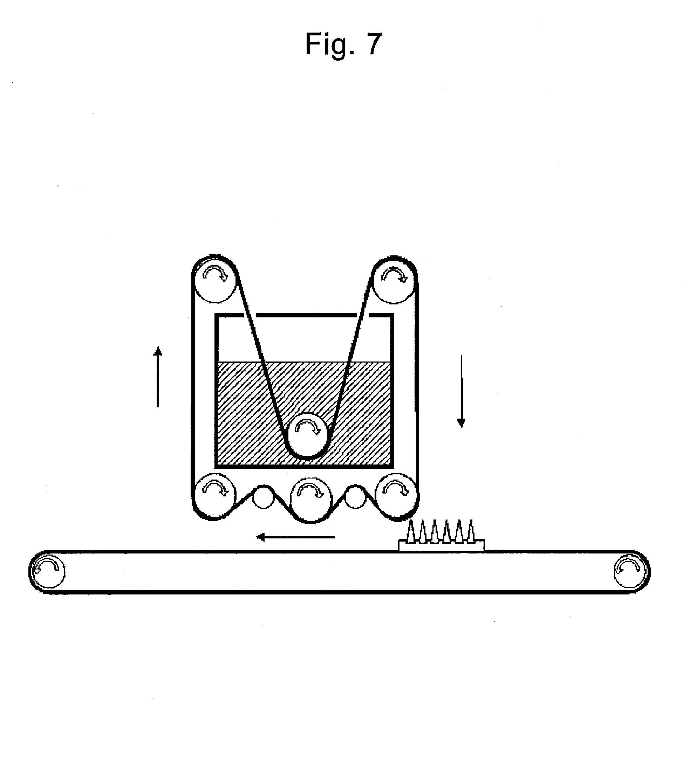

Fig. 7 is a schematic view showing one of the application methods of the present invention, which uses a belt member. The Figure clarifies the positional relationship between the belt and the pin-frog-shaped microneedle when the drug liquid is applied several times.

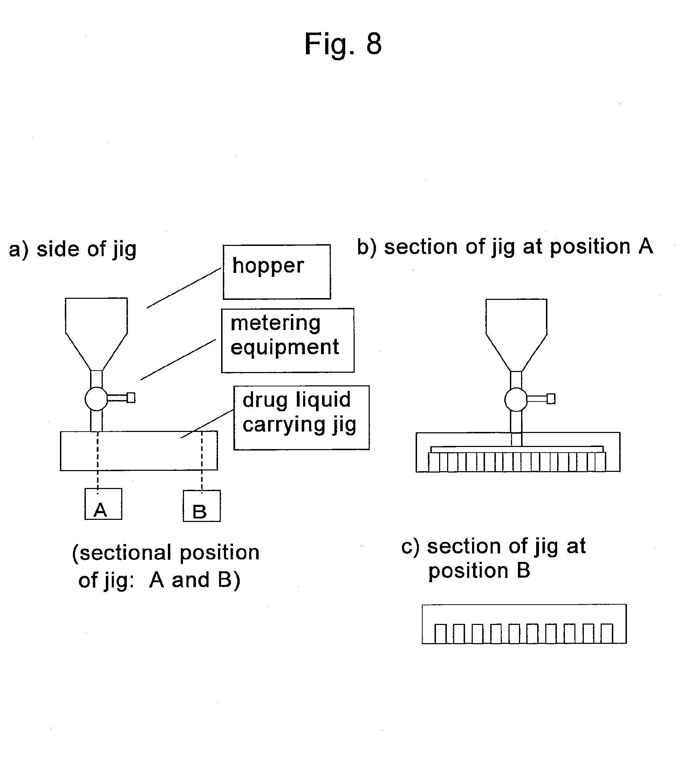

Fig. 8 shows the flat plate-like member to be used in the present invention.

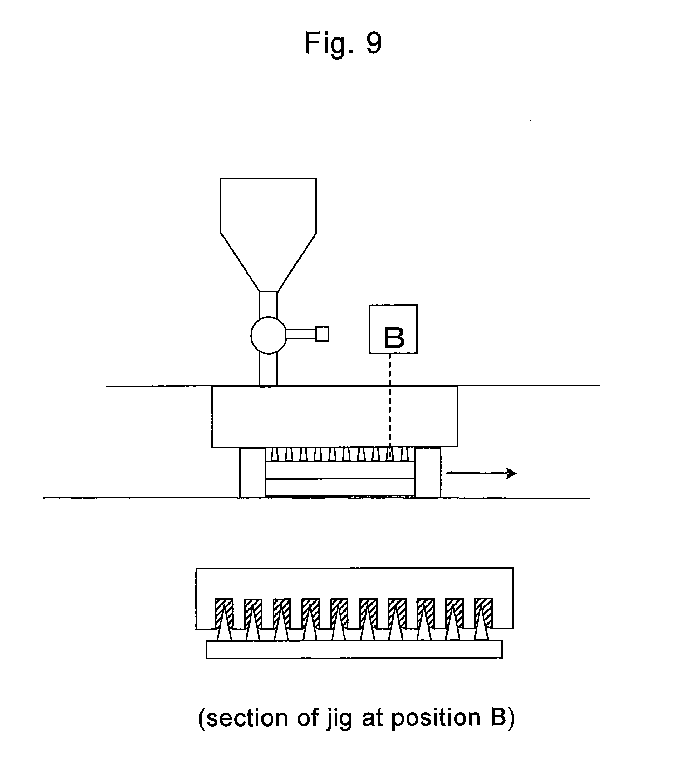

Fig. 9 shows a step following Fig. 5 in the application method of the present invention, which indicates that a pin-frog-shaped microneedle placed on the carrier passes through the grooves.

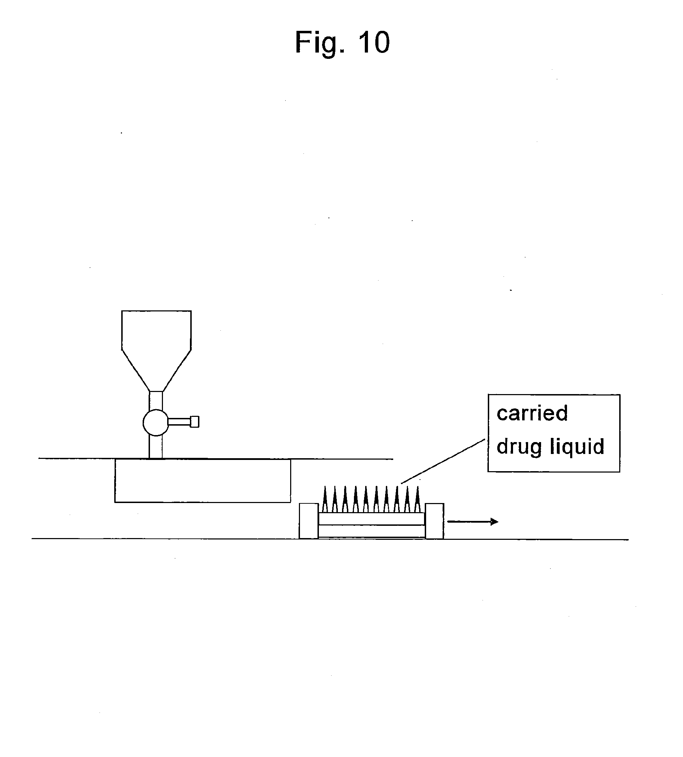

Fig. 10 shows a step following Fig. 9 in the application method of the present invention, which indicates that the carrier passes through the grooves and the drug liquid is applied to the tip portion of the pin-frog-shaped microneedle.
Fig. 11 shows one embodiment of the hourglass-shaped member having a baffle plate placed on both ends of the grooves used in the application method of the present invention.

### [Description of Embodiments]

The first embodiment of the present invention relates to a method of coating a tip portion of a pin-frog-shaped microneedle by using a member having grooves having a cylindrical shape or a cylindrical shape with a missing part.
The member having a cylindrical shape with a missing part refers to a member wherein the surface having the grooves forms a part of the surface of the cylinder. Examples of the cross-sectional shape of the member include a waterwheel shape, a spindle shape, an hourglass shape, a Ginkgo shape and a sector shape.
The first embodiment of the application method of the present invention is explained in detail by referring to the Figures. First, as the member to be used in the present invention, for example, a waterwheel-shaped member is shown in Fig. 4 and an hourglass-shaped member is shown in Fig. 6. The number of the axes of the waterwheel (grooved plate) shown in the Figure is not limited to 4 in the Figure, and can be appropriately determined according to the situation. In addition, no particular problem is caused even when the number of the plates having grooves of the hourglass-shaped member is three rather than two, and can be determined according to the situation.

The number of the grooves in the present invention corresponds to that of the row of small needles of the pin-frog-shaped microneedle to be coated. For example, a desirable number is 5 - 20 grooves. While the depth and width of the grooves can be appropriately determined for the pin-frog-shaped microneedle to be the coating target, the depth of the grooves is, for example, 0.1 - 10 mm, preferably 100 - 600 µm. The width of the grooves is, for example, 100 - 1000 µm, preferably 300 - 500 µm. The pitch width is determined according to that of the pin-frog-shaped microneedle and is, for example, 300 - 1000 µm. The length of the grooves can be appropriately determined and only needs to permit coating of a pin-frog-shaped microneedle. When a member having grooves moves in synchronization with the movement of the microneedle, the length only needs to be the length of the row of the microneedle.
To supply a drug liquid to each groove, the member having grooves is immersed in a drug liquid tank. After filling the drug liquid in each groove, the member is rotated, and the plate with grooves is pulled up from the drug liquid tank. While the drug liquid is maintained in the grooves by surface tension, the redundant drug liquid remains attached to the surface of the plate. To scrape off the drug liquid attached to the surface, the drug liquid on the surface of the grooves is removed with a roller or scraper to uniformly flatten the surface of the drug liquid filled in the grooves.

When the drug liquid has a low viscosity, it is difficult to maintain the drug liquid in the grooves, and the drug liquid flows out from the both ends of the grooves when the grooved plate is pulled out from a drug liquid tank. To prevent or reduce this, a shielding plate (baffle plate) is desirably set in the grooves. For example, when the member is cylindrical such as a roller, a baffle plate as shown in Fig. 1 can be set perpendicular to the grooves. In the case of a cylindrical member with a missing part (hourglass-shaped member), a baffle plate is placed on both ends of the grooves, as shown in Fig. 11, whereby flowing out of the drug liquid can be prevented or reduced.
Then, the member is rotated to horizontally set the plate filled with the drug liquid in the grooves, so that, at this position, a tip portion of the pin-frog-shaped microneedle can pass through the grooves. In this case, the surface plate with grooves moves in synchronization with the movement of the microneedle to speed up the movement of the microneedle. Furthermore, to prevent scattering and evaporation of the drug liquid, it is desirable to place the drug liquid tank and the member in a closed system as shown in Fig. 6 and expose only the plate portion with grooves.

As a material of the member having grooves or plate with grooves of the present invention, a general, commercially available product can be utilized. For example, they are produced using silicon, and metals such as nickel, titanium, stainless steel and the like. The grooves of the plate of the present invention can be produced by, for example, known semiconductor processes. In the semiconductor process, for example, lithography such as X-ray lithography, photolithography and the like, and etching such as ion etching, plasma etching and the like can be used. Preferably, Inductively Coupled Plasma Reactive Ion Etching capable of deeply etching with ion (abbreviated as ICP-RIE) Lithography, sputtering, electro-plating and the like can be used. These techniques are described in books and performed, for example, by referring to and applying the techniques described in "First nano-print technology" (Jun Taniguchi, Kogyo Chosakai Publishing Co., Ltd., 2005), M. Elwenspoek, "Basis of silicon micro processing" (Springer-Verlag Tokyo, 2001) and the like.

The shape of the plate with grooves of the present invention may also be a flat-shaped plate or like the surface of a cylinder. When a flat-shaped plate is used, the movement of the member needs to be discontinued once to facilitate passage of the pin-frog-shaped microneedle through the grooves of the flat-shaped plate. Therefore, the movement of the whole member is discontinued once, and the member needs to be moved to set a new flat-shaped plate to a given position to coat the next microneedle after passage of the microneedle through the flat-shaped plate. On the other hand, when the plate of the present invention has a shape of the surface of a cylinder (e.g., hogback-shaped (semicylindrical-shaped) as shown in Fig. 4 or hourglass-shaped as shown in Fig. 6), the plate surface and the grooves are formed in a curve, rather than linearly, to be a part of a circular arc. Therefore, it is not necessary to stop the whole member once since, for example, the hogback-shaped plate only needs to move to a predetermined position in synchronization with the passage of the microneedle. When the plate of the present invention has the above-mentioned shape of the surface of a cylinder, a drug can be applied to a tip portion of the microneedle while simultaneously moving the microneedle and the plate (member).

The second embodiment of the present invention relates to a method of coating a tip portion of the pin-frog-shaped microneedle by using a belt-shaped member having grooves.
The second embodiment of the coating method of the present invention is explained in detail by referring to Figures. First, as a member to be used for the present invention, the belt-shaped member has the cross-section shown in the lower part of Fig. 4. The number of the grooves on the belt to be used in the present invention corresponds to the number of the rows of small needles of the pin-frog-shaped microneedle to be the coating target. The depth and width of the grooves can be appropriately selected according to the pin-frog-shaped microneedle to be the coating target, and the depth of the grooves is, for example, 0.1 to 10 mm, preferably 100 to 600 µm. The width of the grooves is, for example, 100 to 1000 µm, preferably 300 to 500 µm. The pitch width is determined according to the pitch width of the pin-frog-shaped microneedle and is, for example, 300 to 1000 µm.
To supply a drug liquid to each groove, one end of the belt is immersed in a drug liquid tank. After filling each groove with the drug liquid, the drug liquid naturally reaches an optimal surface tension state by itself on the belt equilibrating with a surface tension. Where necessary, a scraper or roller is set on a drug liquid tank to scrape off the drug liquid attached to the surface of the belt. Furthermore, the drug liquid tank is desirably made a closed system as shown in Fig. 2 to prevent scattering and evaporation of the drug liquid.
As a material of the belt in the present invention, a conventional, commercially available product can be utilized. Examples thereof include materials such as rubber, polyurethane, nylon and the like. In addition, engineering plastic permitting fine processing may also be placed on the belt to facilitate processing of the grooves, and the grooves can be formed on the surface of the belt. Examples of the engineering plastic include polyether ether ketone, polyamide imide, Teflon (registered trade mark), polyacrylic acid, polybutyrene terephthalate, polyimide, polyether sulfone, epoxy resin and the like, and one suitable for the object can be used as appropriate.

As the characteristics of the coating method of the present invention, since the grooves are filled with a drug liquid and the surface tension is strong between the side wall and the drug liquid in the grooves, the drug liquid is closely adhered to the side wall of the grooves. Therefore, the surface of the drug liquid in the grooves of the belt is uniform. As shown in Fig. 3, by immersing the microneedle in the grooves uniformly filled with a drug liquid, the tip portion of the pin-frog-shaped microneedle can be uniformly coated with the drug liquid.
Furthermore, as an example of the method of coating a microneedle using the belt member in the present invention, Fig. 7 can be mentioned. In this example, a tip portion of the microneedle can be coated three times with the drug liquid. By such operation of plural applications, a necessary amount of the drug can be carried on the tip portion of the microneedle.

The third embodiment of the present invention relates to a method of coating a tip portion of a pin-frog-shaped microneedle by using a flat plate-like member having grooves.
The third embodiment of the coating method of the present invention is explained in detail by referring to Figures. First, as the flat plate-like member to be used in the present invention, the member having plural grooves and a drug liquid feeding apparatus as described in Fig. 8 is produced. The number of the grooves in the method of the present invention corresponds to the number of the rows of the pin-frog-shaped microneedle to be the application target, as mentioned above. In the same manner as above, the depth and width of the grooves can be appropriately determined according to the pin-frog-shaped microneedle to be the application target. The length of the grooves can be appropriately determined and only needs to be within the range of 5 to 15 mm.
Furthermore, to equalize the drug liquid feeding amount to each groove, a flow path communicating with each groove is provided. However, when each groove is independent, a drug liquid feeding apparatus is independently provided for each groove. In this case, the flow rate of the drug liquid is controlled to be the same for each groove.
The drug liquid feeding apparatus is constituted with a hopper for storage of the drug liquid and a metering equipment for flowing the drug liquid. The metering equipment is controlled to increase or decrease the drug liquid feeding amount corresponding to the decrease of the drug liquid which is applied to the pin-frog-shaped microneedle during passage through the jig.

Fig. 2 shows a preparation step in the coating method of the present invention. A pin-frog-shaped microneedle is fixed on a transport equipment (carrier). In this case, the row of the small needles of the microneedle is set in parallel with the grooves of the jig, and the tip portions of the small needles are set to pass through the center part of the grooves of the jig.
As shown in Fig. 2, a rectangular-parallelepiped member is placed at the front part in the direction of travel of the carrier. The rectangular-parallelepiped member acts, as shown in Figs. 2 and 5 and Figs. 9 - 10, as a scraper for scratching the jig surface to even out the drug liquid protruding from the jig. Therefore, the member may have any shape as long as it achieves the purpose. In addition, by adjusting the height of this member, the length of the coated area of the tip portion of the microneedle can be determined. Furthermore, as shown in Figs. 2 and 5, and Figs. 9 - 10, a similar rectangular-parallelepiped member can be placed on the rear part of the carrier. Different from the member placed at the front part, it is not required to act as a scraper scratching the surface of the jig, the height of the rear member is desirably the same as or not higher than that of the member at the front part.

In Fig. 5, subsequent to the step of Fig. 2, the rectangular-parallelepiped member placed at the front part in the direction of travel of the carrier advances while scratching the jig surface, whereby the drug liquid part swelling out from the grooves of the jig is pressed forward to uniformly fill the grooves with the drug liquid. In the sectional view of the jig at position B, the surface of the drug liquid in the grooves is not swelling but flat. The tip portion of the microneedle passes through the grooves of the jig thus set, during which the drug liquid is applied.
Fig. 9, subsequent to the step of Fig. 5, shows passage of the microneedle through the grooves of the jig. The sectional view of the jig at position B shows immersion of the tip portion of the microneedle in the grooves. In Fig. 8, the whole microneedle is immersed in the grooves of the jig; however, only a part of the microneedle may be immersed when the length of the grooves is short (the width of the comb-shaped jig is short). In this case, the drug liquid feeding apparatus needs to be controlled to increase the feeding rate of the drug liquid, since the drug liquid in the grooves rapidly decreases while being applied to the tip portion of the microneedle.
After application of the drug liquid to the tip portion of the microneedle by the application methods of Figs. 2 to 4 and Fig. 5, wind such as warm air and the like is blown thereagainst, according to the kind of the solvent of the drug liquid applied, to evaporate the solvent, whereby the drug is adhered to the tip portion of the microneedle. In this case,
in consideration of the stability of the drug and the like, nitrogen gas can be used instead of the air to avoid oxidation of the drug.

As the drug usable in the present invention, various kinds such as low-molecular weight compound, protein, vaccine, antigen, nucleic acid and the like can be used, and preferred are desirably protein, vaccine, antigen and nucleic acid which can express effects with a small dose. Examples of the protein include human growth hormone, insulin, calcitonin, somatostatin, erythropoietin and the like. Examples of the vaccine include influenza vaccine, Lyme disease vaccine, measles vaccine, mumps virus vaccine, varicella vaccine, pneumonia vaccine, pertussis vaccine and the like. Examples of the antigen include antigens such as Bordetella pertussis, Streptococcus pneumoniae, rubella virus, Bacillus of green pus (Pseudomonas) and the like. As the nucleic acid, those usable as DNA vaccine are applicable. Examples thereof include DNA vaccine against viruses such as influenza virus, hepatitis B virus, hepatitis C virus, HIV etc. and the like.

Examples of the solvent of the drug liquid to be used in the present invention include water, alcohol solvents such as methanol, ethanol and the like, organic solvents having a boiling point equivalent to or not higher than that of water, such as acetonitrile, THF, ethyl acetate, chloroform and the like. When water is used, filling the grooves of the jig with the drug liquid may be difficult due to the high surface tension. Therefore, a surfactant and the like can be further added to reduce the surface tension.
The drug liquid can further contain a pH adjuster, an antioxidant and the like to improve stability of the drug. As the surfactant, pH adjuster, antioxidant and the like, general, conventionally-known agents can be used.

The pin-frog-shaped microneedle used in the present invention can be appropriately produced by a known method. For example, it can be produced by the method of WO2008/093679 and the like. As the material of the microneedle, a metal or a resin can be used, with preference given to resin. The resin used here is not particularly limited. Examples of the resin usable in the present invention include aliphatic polyester resins such as polylactic acid, polyglycolic acid, lactic acid-glycolic acid copolymer and the like, polypeptides such as collagen, gelatin and the like, polysaccharides such as hyaluronic acid, chitin, chitosan, maltose, alginate, amylose, agarose, dextran and the like, celluloses such as carboxymethylcellulose, hydroxypropylcellulose and the like, thermoplastic synthetic resins such as poly(ethylene terephthalate), polycarbonate, polypropylene, cycloolefin polymer and the like, silicone resin and the like. Preferable examples of the resin include biodegradable resins represented by aliphatic polyester resins such as polylactic acid, polyglycolic acid, lactic acid-glycolic acid copolymer and the like. Of these, polyglycolic acid resin is one of preferable materials since it has high strength, is highly resistant to impact, and is not easily broken.

While the present invention has been more specifically explained in the above based on the Figures, it is not limited thereby in any way. Those of ordinary skill in the art can practice the present invention by adding various changes and modifications based on the present invention. Such changes and modifications are equivalents of the present invention, and within the scope of the Claims of the present invention.

### Industrial Applicability

The coating method of a pin-frog-shaped microneedle of the present invention is characterized in that the apparatus therefor is simple and convenient, an organic solvent other than water can be used as a drug liquid, and the like. The method is considered to have the highest utility as a method of applying a drug only to a tip portion of a microneedle. This coating method enables mass production of a pin-frog-shaped microneedle coated with a drug on a tip portion thereof.

## Claims

1. A method of coating a tip portion of a pin-frog-shaped microneedle with a drug liquid, comprising
a) setting the following pin-frog-shaped microneedle on a transport equipment (carrier),
said microneedle having needles with a height of 100 to 1000 µm,
said microneedle having needles with a basal portion having a width of 100 to 1000 µm,
b) moving the carrier to have a tip portion of the needles of the pin-frog-shaped microneedle pass through the following grooves filled with a drug liquid,
said grooves having the number and distance corresponding to the row of needles of the microneedle,
said grooves having a depth of 0.1 - 10 mm,
c) moving said carrier in synchronization with and in the same direction as the movement of the member having said grooves, and
d) drying the tip portion of said needles coated with the drug liquid to carry the drug on the tip portion.

2. The method according to claim 1, wherein said member having the grooves is flat plate-like, belt-shaped, cylindrically-shaped or cylindrically-shaped with a missing part.

3. The method according to claim 2, wherein the cylindrically-shaped member with a missing part has a cross-section with a waterwheel shape, a spindle shape , an hourglass shape, a Ginkgo shape (Japanese icho shape) or a sector shape.

4. The method according to any of claims 1 - 3, wherein the surface of said grooves filled with the drug liquid is leveled with a roller or scraper to provide a uniform liquid surface of the grooves.

5. The method according to any of claims 1 - 4, comprising applying and drying the drug liquid several times to carry the drug in a desired amount.

6. The method according to any of claims 1 - 5, wherein the needle of said microneedle has a height of 100 to 600 µm and said grooves have a depth of 100 to 600 µm.

7. The method according to any of claims 1 - 6, wherein said grooves are blocked at the both ends with a shielding plate having the same height as the surface of the grooves.

8. The method according to any of claims 1 - 7, wherein the tip portion of said pin-frog-shaped microneedle is set facing downward in the vertical direction.

9. The method according to any of claims 1 - 8, wherein said pin-frog-shaped microneedle is made of a thermoplastic resin.

10. The method according to any of claims 1 - 9, wherein the thermoplastic resin is a polyglycolic acid resin.
